# EUROPEAN PATENT SPECIFICATION

(11) **EP 2 743 255 B1**
(45) Date of publication and mention of the grant of the patent: **22.06.2016**
(21) Application number: 13197519.5
(22) Date of filing: 16.12.2013
(51) Int. Cl.: C07C 233/18, A61P 25/24, A61K 31/165

(54) **Cocrystal of agomelatine with phosphoric acid**
Co-Kristall von Agomelatin mit Phosphorsäure
Cocristal d'agomélatine avec de l'acide phosphorique

(30) Priority: 17.12.2012 IN CH52722012; 01.11.2013 IN CH49632013
(43) Date of publication of application: 18.06.2014
(73) Proprietor: Dr. Reddy's Laboratories Ltd., 500 034 Andhra Pradesh (IN)
(72) Inventor: Kumar, Vujjini Satish, Andhra Pradesh (IN); Rao, Badarla Venkata Krishna, 521130 Andhra Pradesh (IN); Charyulu, Kandala Sreenadha, 500072 Andhra Pradesh (IN); Praveen, Cherukupally, 502313 Andhra Pradesh (IN); Reddy, Yerraguntla Sesha, 500072 Andhra Pradesh (IN); Dey, Archan, 500090 Andhra Pradesh (IN)
(74) Representative: Bates, Philip Ian

(56) References cited:
- EP-A1- 2 418 195
- CN-A- 102 702 008

## Description

### INTRODUCTION

The present invention relates to cocrystal of agomelatine with phosphoric acid and its method of preparation. Also disclosed is a process for the preparation of crystalline Form I of agomelatine.

The drug compound having the adopted name "agomelatine" can be represented by structural formula (I), and it has a new pharmacological mechanism of action, which combines its melatonin MT₁ and MT₂ agonist properties with a serotonin 5-HT_{2c} antagonist effect. The 5-HT_{2c} receptor is considered a relevant target with regard to antidepressant therapy, as several currently used antidepressant drugs have 5-HT_{2c} receptor antagonist properties.

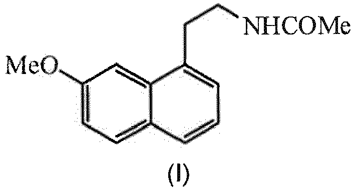

A chemical name for agomelatine is N-[2-(7-methoxy-1-naphthylen-1-yl)ethyl]acetamide and it is the active ingredient in VALDOXAN^{®} tablets, sold for the treatment of major depressive episodes.

U.S. Patent No. 5,225,442 discloses agomelatine, a pharmaceutically acceptable salt thereof, a pharmaceutical composition thereof, and a method for the treatment of a living animal afflicted with treatable disorder of the melatoninergic system, comprising the step of administering to the living animal an effective amount of the compound.

The physicochemical properties endowed by the solid-state structure is a critical parameter in the development of solid dosage forms of pharmaceuticals as these properties can affect the bioavailability, stability and processability of the active pharmaceutical ingredient. It is known that a solid active pharmaceutical ingredient can potentially exist in both amorphous and crystalline forms. It is further known that for a crystalline solid various polymorphs and solvates are possible.

The occurrence of different crystal forms i.e., polymorphism, is a property of some solid compounds. A single molecule may give rise to a variety of polymorphs having distinct crystal structures and physical properties, such as PXRD patterns, IR absorption spectra, melting points (MP), TGA curves, DSC curves, solubilities, stabilities, hygroscopicities and different mechanical properties such as filterability and flowability. The discovery of new polymorphs and solvates of a pharmaceutical active compound provides an opportunity to improve the performance of a drug product in terms of its bioavailability or release profile in vivo, or it may have improved stability or advantageous handling properties. Polymorphism is an unpredictable property of any given compound. This subject has been reviewed in recent articles, including A. Goho, "Tricky Business," Science News, August 21, 2004. In general, one cannot predict whether there will be more than one form for a compound, how many forms will eventually be discovered, or how to prepare any previously unidentified form.

The active pharmaceutical ingredients (APIs) containing an acidic or basic functional group this principle can be exploited by the preparation of various crystalline salts of the active pharmaceutical ingredient to modulate and optimize the physicochemical properties of the obtained crystalline solid for a specific application. The changes in the physicochemical properties resulting from the inclusion of a counter ion in the crystal structure are a consequence of both the molecular structure and properties of the active pharmaceutical molecule and counter ion and the intermolecular interactions between the molecules in the crystal structure. It is therefore possible to change the physicochemical properties of the crystalline solid through the inclusion of different counter ions, giving crystalline salts with different physicochemical properties.

A major limitation of salt formation is that it is inapplicable to neutral APIs. Furthermore, the range of possible counter ions for weakly acidic or weakly basic APIs can be limited by the ionization constant of the acid or base groups on the molecule. Finally, it has been demonstrated that the composition of crystalline molecular salts can be highly unpredictable, particularly with regards to hydrate and solvate formation.

On the other hand, the formation of pharmaceutically acceptable co crystals of active pharmaceutical ingredients provides an alternative approach to the generation of new solid forms of the active substance. In this context a cocrystal, or alternatively co-crystal, is understood to be a binary molecular crystal containing the molecules of the API together with another molecular species in a defined stoichiometric ratio where both components are in their neutral state. In this case the terms "cocrystal" and "co-crystal" are generally understood to be synonymous terms referring to such a system. The second component in the cocrystal (the component other than the active pharmaceutical ingredient) is commonly referred to as a "cocrystal former". Pharmaceutically acceptable cocrystal formers include any molecule considered acceptable as a counter ion for a pharmaceutical salt or known as a pharmaceutical excipient.

A widely accepted definition of a pharmaceutical cocrystal is a crystalline system containing an active pharmaceutical molecule and a cocrystal former that is a solid at ambient temperature and pressure in a defined stoichiometric ratio, although a cocrystal is not limited to containing only two components. The components of the cocrystal are linked by hydrogen bonding and other non-covalent and non-ionic interactions. This definition differentiates co crystals from crystalline solvates, in which case one of the components is a liquid at ambient temperature and pressure.

International Application Publication No. WO 2012/146371 A1 describes cocrystals of agomelatine with citric acid, maleic acid and benzenesulfonic acid. International Application Publication No. WO 2012/046253 A2 describes cocrystal of agomelatine with oxalic acid. International Application Publication No. WO 2012/168665 A1 also describes cocrystals of agomelatine with parahydroxybenzoic acid, citric acid, oxalic acid, gallic acid, maleic acid, malonic acid, glutaric acid, glycolic acid and ketoglutaric acid. The *"*Crystal Growth and Design, 2012, 2226-2233" describes cocrystals of agomelatine with Urea, Glycolic Acid, Isonicotinamide and Methyl 4-Hydroxybenzoate. *"*Crystal Growth and Design, 2011, 466-471*"* describes cocrystals of agomelatine with acetic acid and ethylene glycol.

Still there remains a need for additional cocrystal of agomelatine which can be used in the pharmaceutical formulation.

Further, as stated at column 7, lines 38-42 of U.S. Patent No. 7,250,531: "The prior art EP 0 447 285 and Yous et al. (Journal of Medicinal Chemistry, 1992, 35 (8), 1484 1486*)* allows agomelatine to be obtained in a particular crystalline form which has been described in Tinant et al., (Acta Cryst., 1994, C50, 907 910)."

Journal of Medicinal chemistry 1994, 37, 3231-3239 discloses that agomelatine was obtained as a crystalline solid from a mixture of toluene/hexane in the ration of 2:1 having melting range 109-110°C.

This crystalline form that has been described in the above literature is designated as "Form I" in the literature. A powder X-ray diffraction (PXRD) pattern of Form I is described in International Application Publication No. WO 2011/054917 A1.

International Application Publication Nos. WO 2011/06387 A1, WO 2011/054917 A1, WO 2011/128413 A1 and WO 2012/097764 A1 disclose various processes for the preparation of crystalline Form I of agomelatine. Chinese publications CN 101704763 A and CN 101921205 A also disclose process for the preparation of crystalline Form I of agomelatine.
CN102702008A disclosed agomelatine sulfuric acid complex and EP2418195A1 disclosed hydrogen halide complex of agomelatine.

The processes disclosed in the prior art for the preparation of agomelatine crystalline Form I suffer from one or more drawbacks as reproducibility, stability, less yield, which does not result in an industrially feasible process.

Hence, there is a need to provide a simple, reproducible, and industrially feasible process for the preparation of crystalline Form I of agomelatine.

### SUMMARY

In the first aspect, the application provides a phosphoric acid cocrystal of agomelatine. The phosphoric acid cocrystal of agomelatine may be characterized by a PXRD pattern having peaks at 4.3, 11.0, 13.0, 13.5, 17.4, 20.3, 22.3, 22.5, and 28.0, ±0.2° 2θ. The phosphoric acid cocrystal of agomelatine may be further characterized by a PXRD pattern having peaks at 8.6, 20.0, 20.9, 26.7, and 31.0 ±0.2° 2θ. The phosphoric acid cocrystal of agomelatine may be further characterized by a PXRD pattern having peaks located substantially as shown in Fig.
1. In the second aspect, the application provides a process for the preparation of phosphoric acid cocrystal of agomelatine; comprising the steps of;
   a) providing a solution of agomelatine in a solvent;
   b) adding phosphoric acid to the solution obtained in step a); and
   c) isolating the phosphoric acid cocrystal of agomelatine.

### BRIEF DESCRIPTION OF THE DRAWINGS

Fig. 1 depicts a PXRD pattern of phosphoric acid cocrystal of agomelatine, obtained by the procedure of Example 1.
Fig. 2 depicts a DSC of phosphoric acid cocrystal of agomelatine, obtained by the procedure of Example 1.

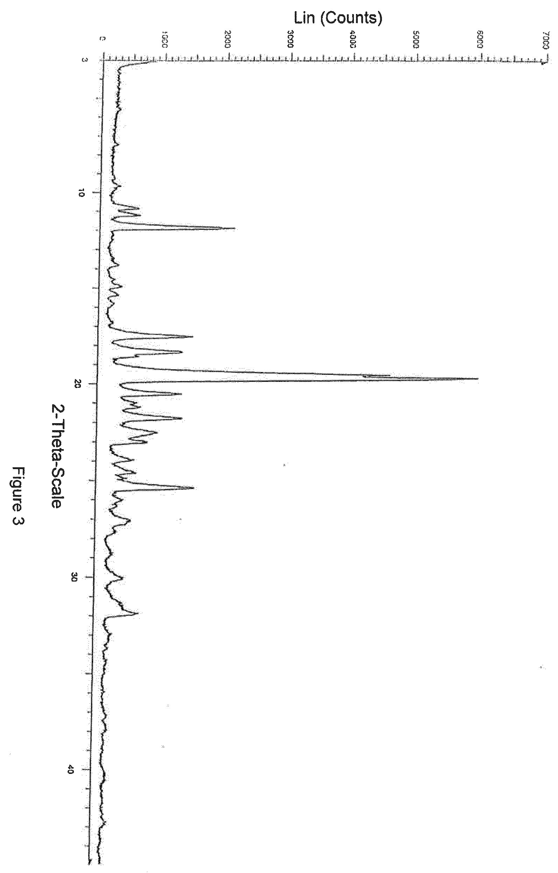

Fig. 3 depicts a PXRD pattern of agomelatine Form I, obtained by the procedure of Reference Example 4.
Fig. 4 depicts a PXRD pattern of phosphoric acid cocrystal of agomelatine, obtained by the procedure of Example 7.
Fig. 5 depicts a PXRD pattern of phosphoric acid cocrystal of agomelatine obtained from example-7 and recorded at 50°C.

### DETAILED DESCRIPTION

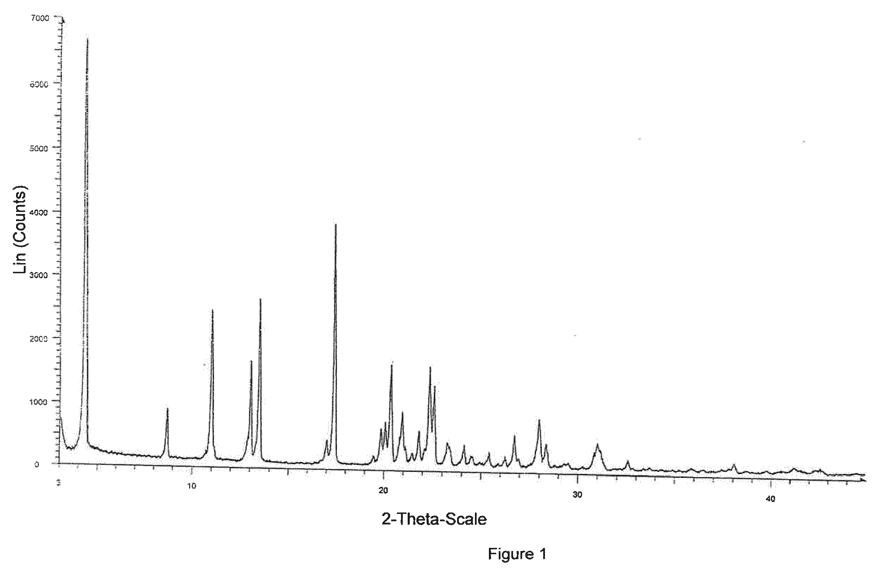

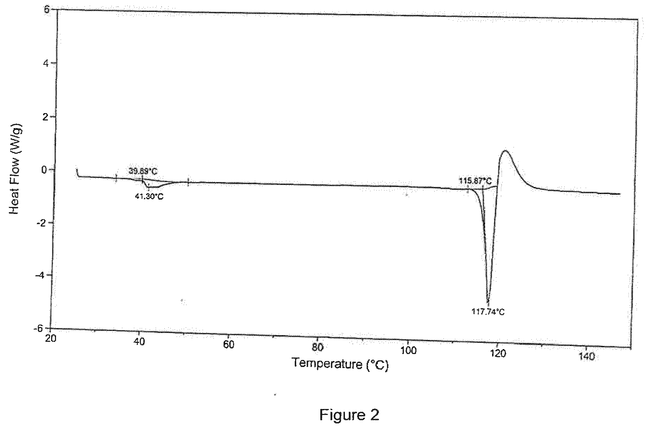

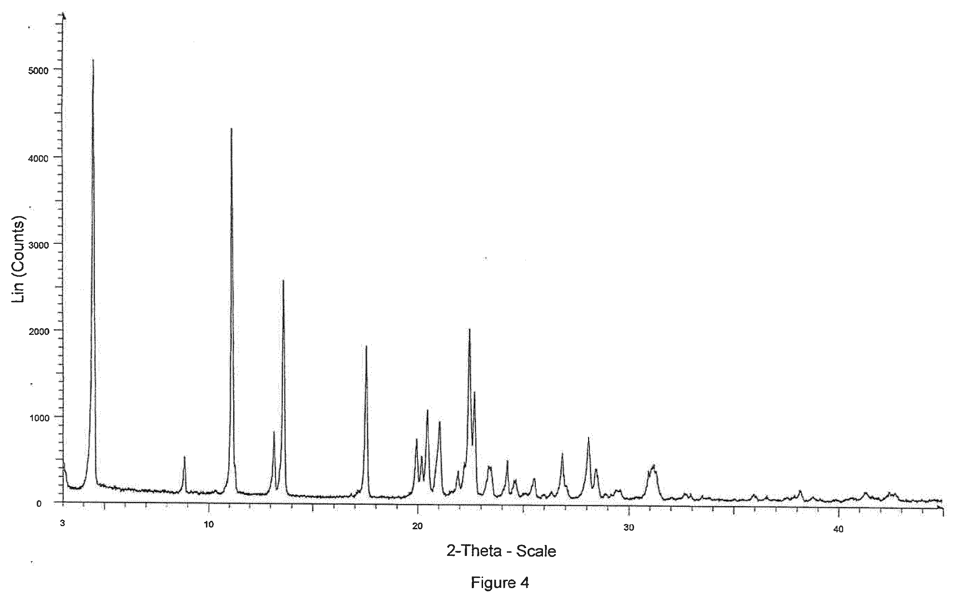

In the first aspect, the application provides a phosphoric acid cocrystal of agomelatine. The phosphoric acid cocrystal of agomelatine may be characterized by a PXRD pattern having peaks at 4.3; 11.0, 13.0, 13.5, 17.4, 20.3, 22.3, 22.5, and 28.0, ±0.2° 2θ. The phosphoric acid cocrystal of agomelatine may be further characterized by a PXRD pattern having peaks at 8.6, 20.0, 20.9, 26.7, and 31.0 ±0.2° 2θ. The phosphoric acid cocrystal of agomelatine may be further characterized by a PXRD pattern having peaks located substantially as shown in Fig. 1. The phosphoric acid cocrystal of agomelatine may be further characterized by differential scanning calorimetry (DSC) thermograph substantially as shown in Fig. 2. The stoichiometric ratio between the agomelatine and phosphoric acid in the phosphoric acid cocrystal of agomelatine may be in the range of 3:1 to 1:3. According to present application the preferred and most suitable stoichiometric ratio is 1:1. The phosphoric acid cocrystal of agomelatine may be further characterized by a PXRD pattern having peaks located substantially as shown in Fig. 4.

In an aspect, the phosphoric acid cocrystal of agomelatine as described herein can be characterized by the following parameters, obtained from the powder diagram obtained using a Panalytical X'pert pro having a 2θ angular range of 3°-80°, a step size of 0.017° and 899.8 s per step:
Monoclinic crystal lattice
Lattice parameters a=22.0925Å, b=4.6809A, c=17.4464A, β=112.356°
Space group: P2₁/C
Number of molecules in the unit cell: 4
Unit cell volume: *V_{unit cell}* = 1668.57 Å³

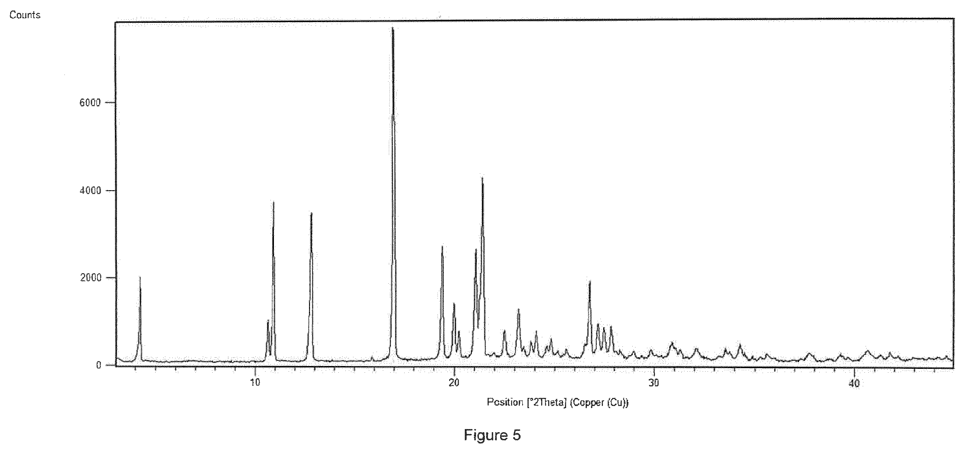

In an aspect, the phosphoric acid cocrystal of agomelatine as described in the present application is studied under variable temperature-PXRD (Anton Paar TTK-450). It is observed that, when the initial sample containing the characteristic 2θ peaks as described herein was heated to 50°C, the peaks at 8.6°, 13.5° and 17.4°± 0.2° 2θ disappeared and also observed the variation in the peaks and intensities of other peaks. The PXRD pattern of phosphoric acid cocrystal of agomelatine upon heating to 50°C can be further characterized by a PXRD pattern having peaks located substantially as shown in Fig. 5. When the heated sample was again cooled to 30°C, the PXRD pattern appeared as a mixture of the original PXRD pattern and the PXRD pattern obtained at 50°C.

In an aspect, the phosphoric acid cocrystal of agomelatine as described in the present application can also be referred as agomelatine phosphoric acid or agomelatine phosphoric acid complex. In an aspect, the word co-crystal and complex as described herein can be used interchangeably.

As used herein, the term "complex" refers to a molecular entity formed by association involving two or more component molecular entities (ionic or uncharged), or by association involving two or more chemical species. The bonding between the components is non-covalent and is normally weaker than covalent bonding. Accordingly, the Agomelatine-phosphoric acid complex described herein is a molecular entity formed by the association between Agomelatine and phosphoric acid. The Agomelatine- phosphoric acid complex may in some embodiments exist as a solid state form that is referred to herein as a co crystal form of an Agomelatine-phosphoric acid complex, or as an Agomelatine phosphoric acid co-crystal, or as a crystalline Agomelatine- phosphoric acid complex.

All of the Agomelatine crystalline forms which were disclosed in the above mentioned publications, except for Form II as disclosed in EP 1564202, were found to be unstable during storage, whereupon they transformed into Form II, or they were found to be not reproducible.

### Stability:

The Agomelatine Phosphoric acid complex of the present invention is reproducible and also stable during storage. For example, at 25°C / 60% relative humidity, or at 30°C / 65% relative humidity, the Agomelatine phosphoric acid complex of the present invention remained stable for at least 6 months.

| Test | Stability at 30°±2°C/RH 65± 5% | | | | |
|---|---|---|---|---|---|
| | Initial | After 1 month | After 2 months | After 3 months | After 6 months |
| Polymorph by PXRD | Crystalline | Same as initial | Same as initial | Same as initial | Same as initial |
| Chemical purity by HPLC | 99.97 | 99.96 | 99.96 | 99.96 | 99.96 |
| Water content (% w/w) (Karl Fischer) | 0.16 | 0.22 | 0.19 | 0.17 | 0.19 |

From the above stability data, the Agomelatine phosphoric acid complex of the present invention is physically and chemically stable for at least 6 months.

In addition, the Agomelatine Phosphoric acid complex exhibits significantly enhanced solubility and enhanced rate of dissolution, compared to the moderately soluble Agomelatine Form II. The enhanced solubility, which is apparently facilitated by the stoichiometric incorporation of the highly soluble Phosphoric acid into the Agomelatine crystal lattice, constitutes an important advantage in formulations.

The Agomelatine Phosphoric acid complex may also improve stability and shelf-life of the API, or give rise to improved processing or handling properties during formulation. The complex may also provide improvements for the final dosage form, for instance by improving the dissolution profile, or bioavailability of the finished product, or by improving the shelf life of the finished dosage form.

In view of the above, Agomelatine Phosphoric acid complex might have superior alternative to Agomelatine Form II.

In the second aspect, the application provides a process for the preparation of phosphoric acid cocrystal of agomelatine; comprising the steps of;
a) providing a solution of agomelatine in a solvent;
b) adding phosphoric acid to the solution obtained in step a); and
c) isolating the phosphoric acid cocrystal of agomelatine.

In an embodiment of step a), agomelatine can be dissolved in any suitable inert solvent. Suitable inert solvents include any solvents that have no adverse effect on the compound and can dissolve to a useful extent. Examples of such solvents include, but are not limited to: ethers, such as diethyl ether, diisopropyl ether, tetrahydrofuran, dioxane, and dimethoxyethane; ketones, such as acetone, methyl ethyl ketone, methyl isobutyl ketone, and diethyl ketone; esters, such as ethyl acetate, propyl acetate, isopropyl acetate, and butyl acetate; alcohols, such as methanol, ethanol, 1-propanol, 2-propanol (isopropyl alcohol), 2-methoxyethanol, 1-butanol, 2-butanol, iso-butyl alcohol, t-butyl alcohol, 2-ethoxyethanol, glycerol, and C₁-C₆ alcohols; nitriles, such as acetonitrile and propionitrile; amides, such as formamide, N,N-dimethylformamide, N,N-dimethylacetamide, N-methyl-2-pyrrolidone, and hexamethylphosphoric triamide; sulfoxides, such as dimethyl sulfoxide; halogenated hydrocarbons, such as dichloromethane and chlorobenzene; aromatic hydrocarbons, such as toluene, ethylbenzene, xylenes; any mixtures of two or more thereof.

In embodiments, a solution of agomelatine can be prepared at any suitable temperatures, such as about 20°C to about the reflux temperature of the solvent used. Mixing may be used to reduce the time required for the dissolution process. Agomelatine solution can also be obtained from reaction of the previous stage during the synthesis of agomelatine. In embodiments, a solution of agomelatine may be filtered to make it clear, free of unwanted particles. In embodiments, the obtained solution may be optionally treated with an adsorbent material, such as carbon and/or hydrose, to remove colored components, etc., before filtration.

In an embodiment of step b), phosphoric acid is added to the agomelatine solution obtained in step a) to obtain phosphoric acid cocrystal of agomelatine. The molar ratio of phosphoric acid to agomelatine can be in the range of 1:3 to 3:1. Preferably the mole ratio of phosphoric acid to agomelatine can be 1:1. In embodiments, the phosphoric acid can be added to the agomelatine solution in solid form (anhydrous) or dissolving the anhydrous form in a solvent or commercially available phosphoric acid solution (assay 85%). The phosphoric acid can be added to the agomelatine solution in one lot or many lots. The phosphoric acid if used in solution form can be added to the agomelatine solution drop wise or in one lot. In embodiments, the phosphoric acid can be added to the agomelatine solution from a temperature about 0°C to the reflux temperature, preferably at the room temperature.

In an embodiment, the precipitation of phosphoric acid cocrystal of agomelatine can be obtained by cooling the solution or suspension obtained in step b). In embodiments, the solution or suspension obtained in step b) can be cooled to room temperature to -10°C preferably about 0°C to 15°C. In embodiments, the slurry comprising precipitated product can be maintained at any suitable temperatures, such as from about room temperature to about 0°C. In general, yields of the product will be improved by maintaining the reaction mass at lower temperatures that are above the freezing point of the solvents and/or by increasing the solute content of the solution. In embodiments, the slurry comprising precipitated phosphoric acid cocrystal of agomelatine can be maintained for about 10 minutes to about 10 hours, or longer.

In embodiments of step c), the precipitated phosphoric acid cocrystal of agomelatine is isolated. In embodiments, solid phosphoric acid cocrystal of agomelatine can be isolated using any techniques, such as decantation, filtration by gravity or suction, centrifugation, or the solvent can be evaporated from the mass to obtain the desired product, and optionally the solid can be washed with a solvent, such as an anti-solvent, to reduce the amount of entrained impurities. In embodiments, the phosphoric acid cocrystal of agomelatine can be isolated by filtration of the slurry containing precipitated product.

In embodiments, phosphoric acid cocrystal of agomelatine that is isolated can be dried at suitable temperatures, and atmospheric or reduced pressures, for about 1-50 hours, or longer, using any types of drying equipment, such as a tray dryer, vacuum oven, air oven, fluidized bed dryer, spin flash dryer, flash dryer, and the like. Drying temperatures and times will be sufficient to achieve desired product purity.

The Agomelatine phosphoric acid cocrystal can be prepared, for example, by a process comprising co-milling Agomelatine with phosphoric acid until the molecular complex of Agomelatine and phosphoric acid is formed. The crystalline Agomelatine-phosphoric acid complex may also be prepared by slow evaporation of a saturated solution of the Agomelatine phosphoric acid complex in a suitable solvent at ambient temperature.

The above mentioned Agomelatine phosphoric acid complex can inter alia be used to prepare Agomelatine polymorphs, for example, Agomelatine Form II, as described in EP 1564202, or it can be used to prepare other complexes of Agomelatine.

In embodiments, phosphoric acid cocrystal of agomelatine according to the present application can be substantially pure having a chemical purity greater than about 99%, or greater than about 99.5%, or greater than about 99.9%, by weight, as determined using high performance liquid chromatography (HPLC). Phosphoric acid cocrystal of agomelatine according to the present application can be chemically pure having purity greater than about 99.5% and containing no single impurity in amounts greater than about 0.15%, by HPLC. Phosphoric acid cocrystal of agomelatine according to the present application can be chemically pure agomelatine having purity greater than about 99.8% and containing no single impurity in amounts greater than about 0.1%, by HPLC.

In embodiments, the phosphoric acid cocrystal of agomelatine according to the present application can be milled or micronized by any process known in the art, such as ball milling, jet milling, wet milling *etc.,* to produce desired particle sizes and particle size distributions.

In embodiments, phosphoric acid cocrystal of agomelatine according to the present application has a particle size distribution wherein: mean particle size is less than about 200 µm or less than about 100 µm; or less than about 50 µm; d(0.1) is less than about 150 µm or less than about 25 µm; or less than about 15 µm; d(0.5) is less than about 200 µm or less than about 25 µm; and d(0.9) is less than about 250 µm or less than about 50 µm. In another aspect of the application phosphoric acid cocrystal of agomelatine obtained by the processes herein described, having a mean particle size of about less than about 100 µm.

In embodiments, phosphoric acid cocrystal of agomelatine herein as described, having bulk density of about 0.1 g/mL to 1.0 g/mL, preferably from about 0.1 g/mL to 0.6 g/mL and tapped bulk density 0.1 g/mL to 1.0 g/mL, preferably from about 0.2 g/mL to 0.8 g/mL.

In an aspect, the application provides packing conditions for Agomelatine phosphoric acid co-crystal/complex comprises of the following steps;
a) packing the Agomelatine phosphoric acid co-crystal/complex into a clear low-density polyethylene bag which is flushed with nitrogen; tie it with tag/plastic strip;
b) placing the above polyethylene bag in a black polyethylene bag and tie with tag/plastic strip;
c) placing the above polyethylene bag obtained in step b) in a triple laminated bag along with silica pouch (which is previously dried for 3 hours under vacuum at 105°C) and seal it with 4-liner sealer;
d) placing the above bag obtained in step c) in HDPE container and sealing the container;
e) storing the container in controlled environment chamber. The application further discloses a process for the preparation of crystalline Form I of agomelatine; comprising the steps of;
   a) providing a solution of agomelatine in isopropyl alcohol or isopropyl acetate;
   b) adding the solution obtained in step a) to a pre-cooled flask and stirring to allow the precipitation;
   c) adding the pre-cooled n-heptane to the slurry obtained in step b); and
   d) isolating the crystalline form I of agomelatine.

In an embodiment of step a), agomelatine can be dissolved in isopropyl alcohol or isopropyl acetate to provide a solution. In embodiments, a solution of agomelatine can be prepared at any suitable temperatures, such as from about room temperature to about the reflux temperature of the solvent used. Preferably the agomelatine solution can be obtained by dissolving agomelatine in isopropyl alcohol or isopropyl acetate on heating. Mixing may be used to reduce the time required for the dissolution process. Agomelatine solution can also be obtained from reaction mixtures of the previous stage during the synthesis of agomelatine. In an embodiment, a solution of Agomelatine may be filtered to make it clear, free of undissolved particles. In embodiments, the obtained solution may be optionally treated with a decolorizing agent or an adsorbent material, such as carbon and/or hydrose, to remove colored components, *etc.,* before filtration.

In an embodiment of step b), the solution of agomelatine obtained in step a) can be added to a pre-cooled flask. In embodiments, the empty flask can be cooled between -25°C to -5°C before the addition of the agomelatine solution. The precipitation can occur immediately or while stirring at the same temperature. The temperature may be raised during or after the addition of agomelatine solution and cooling can be controlled such that it may not raise more than 10°C. The precipitate can be further stirred for about 5 minutes to 15 minutes at the same temperature.

In an embodiment of step c), a pre-cooled n-heptane is added to the slurry obtained in step b) at -10°C to 10°C, preferably -10°C to 0°C. In embodiments, n-heptane is cooled to -10°C before adding to the slurry. In embodiments, pre-cooled n-heptane is added to the slurry in one lot or many lots. In embodiments, pre-cooled n-heptane is added to the slurry in one lot. The slurry obtained after the addition of n-heptane can be stirred for a period of about 15 minutes to 5 hours, or longer at -10°C to 10°C. In embodiments, the slurry obtained in step c) can be maintained with or without stirring at -10°C to 10°C for 15 minutes to 5 hours or longer. In embodiments, the ratio of solvent to anti-solvent (n-heptane) can be between 1: 5 to 1:30. In an embodiment of step c) any other suitable anti-solvents other than n-heptane can also be added to the slurry obtained in step b). The anti-solvents that can be used but are not limited to hydrocarbons such as n-pentane, n-hexane, cyclohexane, ethers such as diisopropylether, methyl tertiary butylether etc.

In an embodiment of step d), the precipitated crystalline form I of agomelatine can be isolated using any techniques, such as decantation, filtration by gravity or suction, centrifugation, or the solvent can be evaporated from the mass to obtain the desired product, and optionally the solid can be washed with a solvent or anti-solvent, used for the crystallization to reduce the amount of entrained impurities in the product. In embodiments, crystalline form I of agomelatine can be isolated by filtration.

In embodiments, crystalline form I of agomelatine that is isolated can be dried at suitable temperatures such as room temperature to about 80°C under atmospheric or reduced pressures, for about 10 minutes to about 50 hours, or longer, using any types of drying equipment, such as a tray dryer, vacuum oven, air oven, fluidized bed dryer, spin flash dryer, flash dryer, or the like.

In embodiments, crystalline Form I of agomelatine according to the present application can be substantially pure having a chemical purity greater than about 99%, or greater than about 99.5%, or greater than about 99.9%, by weight, as determined using high performance liquid chromatography (HPLC). Crystalline Form I of agomelatine according to the present application can be chemically pure having purity greater than about 99.5% and containing no single impurity in amounts greater than about 0.15%, by HPLC. Crystalline Form I of agomelatine according to the present application can be chemically pure agomelatine having purity greater than about 99.8% and containing no single impurity in amounts greater than about 0.1%, by HPLC.

The crystalline form I of agomelatine according to the present application can be milled or micronized by any process known in the art, such as ball milling, jet milling, wet milling *etc.,* to produce desired particle sizes and particle size distributions. In embodiments, agomelatine which is used as the starting material in the present application can be prepared by any method, including methods known in the art. In embodiments, agomelatine which is used as the starting material can be in any known or unknown crystalline form, anhydrous form, hydrated form, solvate or amorphous form.

In another aspect, the application provides a pharmaceutical composition containing a therapeutically effective amount of phosphoric acid cocrystal/complex of agomelatine together with one or more pharmaceutically acceptable excipients.

The pharmaceutical compositions comprising phosphoric acid cocrystal of agomelatine of the invention together with one or more pharmaceutically acceptable excipients may be formulated as: solid oral dosage forms, such as, but not limited to: powders, granules, pellets, tablets, and capsules; liquid oral dosage forms such as but not limited to syrups, suspensions, dispersions, and emulsions; and injectable preparations such as, but not limited to, solutions, dispersions, and freeze-dried compositions. Formulations may be in the form of immediate release, delayed release or modified release. Further, immediate release compositions may be conventional, dispersible, chewable, mouth dissolving, or flash melt preparations, and modified release compositions may comprise hydrophilic or hydrophobic, or combinations of hydrophilic and hydrophobic, release rate-controlling substances to form matrix or reservoir systems, or combinations of matrix and reservoir systems. The compositions may be prepared using any one or more of techniques such as direct blending, dry granulation, wet granulation, and extrusion and spheronization. Compositions may be presented as uncoated, film coated, sugar coated powder coated, enteric coated, or modified release coated.

Pharmaceutically acceptable excipients that are useful in the present application include, but are not limited to, any one or more of: diluents such as starches, pregelatinized starches, powdered cellulose, microcrystalline cellulose, dicalcium phosphate, tricalcium phosphate, mannitol, sorbitol, sugar, and the like; binders such as acacia, guar gum, tragacanth, gelatin, polyvinylpyrrolidones, hydroxypropyl celluloses, hydroxypropyl methylcelluloses, pregelatinized starches, and the like; disintegrants such as starches, sodium starch glycolate, pregelatinized starches, crospovidones, croscarmellose sodium, colloidal silicon dioxide, and the like; lubricants such as stearic acid, magnesium stearate, zinc stearate, and the like; glidants such as colloidal silicon dioxide and the like; solubility or wetting enhancers such as anionic, cationic, and neutral surfactants; complex forming agents such as various grades of cyclodextrins and resins; and release rate controlling agents such as hydroxypropyl celluloses, hydroxymethyl celluloses, hydroxypropyl methylcelluloses, ethylcelluloses, methylcelluloses, various grades of methyl methacrylates, waxes, and the like. Other pharmaceutically acceptable excipients that are useful include, but are not limited to, film-formers, plasticizers, colorants, flavoring agents, sweeteners, viscosity enhancers, preservatives, antioxidants, and the like.

X-ray powder diffraction patterns described herein were generated using a Bruker AXS D8 Advance powder X-ray diffractometer, with a copper K-alpha radiation source. Generally, a diffraction angle (2θ) in powder X-ray diffractometry may have an error in the range of ±0.2°. Therefore, the aforementioned diffraction angle values should be understood as including values in the range of ± 0.2°. Accordingly, the present invention includes not only crystals whose peak diffraction angles in powder X-ray diffractometry completely coincide with each other, but also crystals whose peak diffraction angles coincide with each other with an error of ± 0.2°. Therefore, in the present specification, the phrase "having a diffraction peak at a diffraction angle (2θ) ±0.2° of 4.3°" means "having a diffraction peak at a diffraction angle (2θ) of 4.1 to 4.5°. Although the intensities of peaks in the x-ray powder diffraction patterns of different batches of a compound may vary slightly, the peak relationships and the peak locations are characteristic for a specific polymorphic form. The relative intensities of the PXRD peaks can vary somewhat, depending on factors such as the sample preparation technique, crystal size distribution, various filters used, the sample mounting procedure, and the particular instrument employed. Moreover, instrumental variation and other factors can slightly affect the 2-theta values. Therefore, the term "substantially" in the context of PXRD is meant to encompass that peak assignments can vary by plus or minus about 0.2°. Moreover, new peaks may be observed or existing peaks may disappear, depending on the type of the machine or the settings (for example, whether a filter is used or not).

Differential Scanning Calorimetry (DSC) analysis was done using a TA Q1000 DSC. The crucible was crimped and punched prior to analysis. The weight of the samples was about 2-5 mg; the samples were scanned at a rate of 10° C/min from 40°C to 150°C. The oven was constantly purged with nitrogen gas at a flow rate of 50 mL/min.

### DEFINITIONS

As used throughout herein, the term room temperature refers to a temperature of from 18°C to 28°C., preferably 20°C to 25°C.

As used throughout herein, the term pre-cooled refers to a temperature from -20°C to 10°C, preferably -10°C to 0°C.

A "decolorizing agent" removes colored impurities by adsorption on the surface of the "decolorizing agent" particles. Examples of a "decolorizing agent" include, decolorizing carbon, activated alumina, activated clay, or silica gel.

Certain specific aspects and embodiments of the present application will be explained in greater detail with reference to the following examples, which are provided only for purposes of illustration and should not be construed as limiting the scope of the application in any manner. Variations of the described procedures, as will be apparent to those skilled in the art, are intended to be within the scope of the present application.

### EXAMPLES

### Example-1: Preparation of phosphoric acid cocrystal of Agomelatine

Agomelatine (10 g) and ethyl acetate (50 mL) were charged into a round bottom flask at 26°C and stirred to get a mixture. Anhydrous orthophosphoric acid (4.0 g) was charged to the mixture at 28°C and stirred to get clear solution. The slurry containing precipitated product was stirred for 30 minutes at 28°C and further stirred for 30 minutes at 5°C. Separated solid was collected by filtration, washed with ethyl acetate (10 mL) and dried at 54°C for 3 hours to obtain the title compound. Yield: 9.8 g,

### Example-2: Preparation of phosphoric acid cocrystal of Agomelatine

Agomelatine (10 g) and ethyl acetate (50 mL) were charged into a round bottom flask at 28°C and stirred to get a mixture. An aqueous solution of orthophosphoric acid (2.8 mL, assay 85%) was charged to the mixture at 28°C and stirred to get clear solution. The slurry containing precipitated product was stirred for 30 minutes at 28°C and further stirred for 30 minutes at 9°C. Separated solid was collected by filtration, washed with ethyl acetate (5 mL) and dried at 59°C for 3 hours to obtain the title compound. Yield: 9.2 g, Purity by HPLC: 99.98%.

### Example-3: Preparation of Agomelatine phosphoric acid cocrystal/complex

Agomelatine (20 g) and ethyl acetate (100 mL) were charged into a round bottom flask at 24°C and stirred to get a mixture. An aqueous solution of orthophosphoric acid (5.6 mL, assay 85%) was charged to the mixture at 24°C and stirred to get clear solution. The slurry containing precipitated product was stirred for 1 hour at 28°C and further stirred for 1 hour at 5°C. Separated solid was collected by filtration and washed with ethyl acetate (20 mL) and dried at 57°C for 4 hours to obtain the title compound. Yield: 22.0 g, Purity by HPLC: 99.99%.

### Reference Example-4:

### Preparation of agomelatine Form I

Agomelatine (50 g) and isopropyl alcohol (150 mL) were charged into a round bottom flask at 26°C. The mixture was heated to 50°C to obtain a clear solution. The hot clear solution was added to a pre-cooled (at -10°C) round bottom flask and stirred to obtain slurry. Pre-cooled n-heptane (1500 mL, at -10°C) was added to the flask containing the slurry and stirred for 30 minutes at -10°C. Separated solid was collected by filtration, washed with pre-cooled n-heptane (50 mL), and dried under vacuum at 37°C for 4 hours to obtain the title compound. Yield: 42.5 g.

### Reference Example 5:

### Preparation of agomelatine Form I

Agomelatine (10 g) and isopropyl acetate (30 mL) were charged into a round bottom flask at 28°C. The mixture was heated to 50°C to obtain a clear solution. The hot clear solution was added to a pre-cooled (at -10°C) round bottom flask and stirred to obtain slurry. Pre-cooled n-heptane (300 mL, at -10°C) was added to the flask containing the slurry and stirred for 30 minutes at -10°C. Separated solid was collected by filtration, washed with pre-cooled n-heptane (10 mL) and dried under vacuum at 35°C for 3 hours to obtain the title compound. Yield: 8.0 g.

### Reference Example 6:

### Preparation of agomelatine Form I

Agomelatine (5.0 g) and isopropyl alcohol (100 mL) were charged into a round bottom flask at 28°C. The mixture was heated to 45°C to get a clear solution. The solution was cooled to -20°C and water (300 mL) added to the solution and stirred for 20 minutes at -5°C. Separated solid was collected by filtration, washed with water (10 mL) and dried under vacuum at 40°C for 3 hours to obtain the title compound. Yield: 4.4 g.

### Example-7: Preparation of Agomelatine phosphoric acid complex/cocrystal

Agomelatine (50 g) and ethyl acetate (350 mL) were charged into a round bottom flask at 30°C and the obtained mixture was heated to 55°C to get a clear solution. The solution was filtered through micron filter and washed with ethyl acetate (100 mL). The solution of phosphoric acid (obtained by dissolving 22.4 g phosphoric acid in 150 mL of ethyl acetate) was added to the agomelatine solution as obtained above slowly in about 45 minutes at 30°C. The slurry containing precipitated product was stirred for 30 minutes at 30°C and further cooled to 5°C and maintained for 30 minutes. The precipitated product was collected by filtration, washed with ethyl acetate (50 mL) and dried at 35°C for 5 hours to obtain the title compound. Yield: 63.2 g; HPLC purity: 99.96%. The PXRD pattern is according to the Figure-4.

A sample of the above obtained compound having the PXRD pattern as shown in Figure-4 was subjected to variable temperature-PXRD (Anton Paar TTK-450). The PXRD pattern of phosphoric acid cocrystal of agomelatine upon heating to 50°C is shown in Figure-5.

## Claims

1. Agomelatine phosphoric acid cocrystal.

2. The Agomelatine phosphoric acid cocrystal of claim 1, wherein the phosphoric acid and Agomelatine molecules are present at an equimolar ratio (1:1).

3. The Agomelatine phosphoric acid cocrystal of claim 1 to 2, **characterized by** a PXRD pattern having peaks at 4.3, 11.0, 13.0, 13.5, 17.4, 20.3, 22.3, 22.5, and 28.0, ±0.2° 2θ.

4. The Agomelatine phosphoric acid cocrystal of claim 1 to 2, further **characterized by** a PXRD pattern having peaks at 8.6, 20.0, 20.9, 26.7, and 31.0 ±0.2° 2θ.

5. A process for preparing Agomelatine phosphoric acid cocrystal of claim 1 to 4, comprising the steps of;
a) providing a solution of agomelatine in a solvent;
b) adding phosphoric acid to the solution obtained in step a); and
c) isolating the agomelatine phosphoric acid.

6. The process as claimed in claim 5, wherein phosphoric acid used in step b) can be in solid form or in liquid form.

7. A process according to claim 5 for preparing Agomelatine phosphoric acid cocrystal of claim 1 to 4, comprising the steps of;
a) providing a solution of agomelatine in ethyl acetate;
b) adding phosphoric acid to the solution obtained in step a); and
c) isolating the agomelatine phosphoric acid cocrystal.

8. A pharmaceutical composition comprising the Agomelatine phosphoric acid cocrystal according to any one of claims 1 to 4, and at least one pharmaceutical acceptable excipient.

9. Use of Agomelatine phosphoric acid cocrystal according to any one of claims 1 to 4 for the manufacture of medicament.

10. A process for preparing a pharmaceutical composition comprising combining the Agomelatine phosphoric acid cocrystal according to any one of claims 1 to 4, and at least one pharmaceutical acceptable excipient.

11. The Agomelatine phosphoric acid cocrystal according to any one of claims 1 to 4, or the pharmaceutical composition of claim 8 for use as a medicament.

12. The Agomelatine phosphoric acid cocrystal according to any one of claims 1 to 4, or the pharmaceutical composition of claim 8 for use in the treatment of depression.

## Patentansprüche

1. Agomelatin-Phosphorsäure-Co-Kristall.

2. Agomelatin-Phosphorsäure-Co-Kristall nach Anspruch 1, wobei die Phosphorsäure- und Agomelatin-Moleküle in einem äquimolaren Verhältnis (1:1) vorliegen.

3. Agomelatin-Phosphorsäure-Co-Kristall nach Anspruch 1 bis 2, **gekennzeichnet durch** ein PXRD-Muster mit Spitzen bei 4,3, 11,0, 13,0, 13,5, 17,4, 20,3, 22, 3, 22, 5 und 28,0 ± 0,2° 2θ.

4. Agomelatin-Phosphorsäure-Co-Kristall nach Anspruch 1 bis 2, ferner **gekennzeichnet durch** ein PXRD-Muster mit Spitzen bei 8,6, 20,0, 20,9, 26,7 und 31,0 ± 0,2° 2θ.

5. Verfahren zur Herstellung eines Agomelatin-Phosphorsäure-Co-Kristalls nach Anspruch 1 bis 4, das die folgenden Schritte beinhaltet:
a) Bereitstellen einer Lösung von Agomelatin in einem Lösungsmittel;
b) Zugeben von Phosphorsäure zu der in Schritt a) erhaltenen Lösung; und
c) Isolieren der Agomelatin-Phosphorsäure.

6. Verfahren nach Anspruch 5, wobei die in Schritt b) benutzte Phosphorsäure in fester Form oder in flüssiger Form vorliegen kann.

7. Verfahren nach Anspruch 5 zur Herstellung eines Agomelatin-Phosphorsäure-Co-Kristalls nach Anspruch 1 bis 4, das die folgenden Schritte beinhaltet:
a) Bereitstellen einer Lösung von Agomelatin in Ethylacetat;
b) Zugeben von Phosphorsäure zu der in Schritt a) enthaltenen Lösung; und
c) Isolieren des Agomelatin-Phosphorsäure-Co-Kristalls.

8. Pharmazeutische Zusammensetzung, die das Agomelatin-Phosphorsäure-Co-Kristall nach einem der Ansprüche 1 bis 4 und wenigstens einen pharmazeutisch akzeptablen Exzipienten enthält.

9. Verwendung eines Agomelatin-Phosphorsäure-Co-Kristalls nach einem der Ansprüche 1 bis 4 für die Herstellung eines Medikaments.

10. Verfahren zur Herstellung einer pharmazeutischen Zusammensetzung, das das Kombinieren des Agomelatin-Phosphorsäure-Co-Kristalls nach einem der Ansprüche 1 bis 4 und von wenigstens einem pharmazeutisch akzeptablen Exzipienten beinhaltet.

11. Agomelatin-Phosphorsäure-Co-Kristall nach einem der Ansprüche 1 bis 4 oder pharmazeutische Zusammensetzung nach Anspruch 8 zur Verwendung als Medikament.

12. Agomelatin-Phosphorsäure-Co-Kristall nach einem der Ansprüche 1 bis 4 oder pharmazeutische Zusammensetzung nach Anspruch 8 zur Verwendung bei der Behandlung von Depression.

## Revendications

1. Cocristal d'agomélatine et d'acide phosphorique.

2. Cocristal d'agomélatine et d'acide phosphorique selon la revendication 1, dans lequel les molécules d'acide phosphorique et d'agomélatine sont présentes dans un rapport équimolaire (1:1).

3. Cocristal d'agomélatine et d'acide phosphorique selon la revendication 1 ou 2, **caractérisé par** un profil XRD de poudre ayant des pics à 4,3, 11,0, 13,0, 13,5, 17,4, 20,3, 22,3, 22,5 et 28,0, ± 0,2° 2θ.

4. Cocristal d'agomélatine et d'acide phosphorique selon la revendication 1 ou 2, **caractérisé en outre par** un profil XRD de poudre ayant des pics à 8,6, 20,0, 20,9, 26,7, et 31,0 ± 0,2° 2θ.

5. Processus de préparation d'un cocristal d'agomélatine et d'acide phosphorique selon les revendications 1 à 4, comprenant les étapes consistant à :
a) fournir une solution d'agomélatine dans un solvant ;
b) ajouter de l'acide phosphorique à la solution obtenue à l'étape a) ; et
c) isoler l'acide phosphorique de l'agomélatine.

6. Processus selon la revendication 5, dans lequel l'acide phosphorique utilisé à l'étape b) peut être sous forme solide ou sous forme liquide.

7. Processus selon la revendication 5 pour préparer un cocristal d'agomélatine et d'acide phosphorique selon les revendications 1 à 4, comprenant les étapes consistant à :
a) fournir une solution d'agomélatine dans de l'éthyle acétate ;
b) ajouter de l'acide phosphorique à la solution obtenue à l'étape a) ; et
c) isoler le cocristal d'agomélatine et d'acide phosphorique.

8. Composition pharmaceutique comprenant le cocristal d'agomélatine et d'acide phosphorique selon l'une quelconque des revendications 1 à 4, et au moins un excipient pharmaceutiquement acceptable.

9. Utilisation d'un cocristal d'agomélatine et d'acide phosphorique selon l'une quelconque des revendications 1 à 4 pour la fabrication d'un médicament.

10. Processus de préparation d'une composition pharmaceutique comprenant la combinaison du cocristal d'agomélatine et d'acide phosphorique selon l'une quelconque des revendications 1 à 4, et d'au moins un excipient pharmaceutiquement acceptable.

11. Cocristal d'agomélatine et d'acide phosphorique selon l'une quelconque des revendications 1 à 4, ou composition pharmaceutique selon la revendication 8 destinée à être utilisée comme médicament.

12. Cocristal d'agomélatine et d'acide phosphorique selon l'une quelconque des revendications 1 à 4, ou composition pharmaceutique selon la revendication 8 destinée à être utilisée dans le traitement de la dépression.
